# EUROPÄISCHE PATENTANMELDUNG

(11) **EP 3 872 029 A1**
(43) Veröffentlichungstag der Anmeldung: **01.09.2021**
(21) Anmeldenummer: 20020169.7
(22) Anmeldetag: 09.04.2020
(51) Int. Cl.: C01B 3/38, C07C 29/151, C25B 1/04, C25B 1/34

(54) **VERFAHREN UND ANLAGE ZUR HERSTELLUNG EINES SYNTHESEPRODUKTS**

(30) Priorität: 29.02.2020 DE 102020001330
(71) Anmelder: Linde GmbH, 82049 Pullach (DE)
(72) Erfinder: Schödel, Nicole, 81477 München (DE); Behrens, Axel, 80689 München (DE); Schmaderer, Harald, 82515 Wolfratshausen (DE); Peschel, Andreas, 82515 Wolfratshausen (DE); Hoffmann, Stefan, 82049 Pullach (DE)
(74) Vertreter: Fischer, Werner

(57) **Zusammenfassung**

Die Erfindung betrifft ein Verfahren (100) zur Herstellung eines oder mehrerer Syntheseprodukte, bei dem ein Methan enthaltender Reformierungseinsatz unter Erhalt eines Synthesegases einer Reformierung (10) unterworfen wird, bei dem zumindest ein Teil des Synthesegases zur Bereitstellung eines Syntheseeinsatzes verwendet wird, und bei dem zumindest ein Teil des Syntheseeinsatzes unter Erhalt des Produkts einer Synthese (20) unterworfen wird. Es ist dabei erfindungsgemäß vorgesehen, dass zumindest in einem ersten Betriebsmodus in den Reformierungseinsatz, die Reformierung (10) und/oder den Syntheseeinsatz Kohlendioxid zugegeben wird, dass zumindest in dem ersten Betriebsmodus das Synthesegas in der Reformierung (10) mit einem Wasserstoffgehalt, der geringer ist als ein stöchiometrischer Bedarf in der Synthese (20) und mit einem Kohlenmonoxidgehalt, der größer ist als ein stöchiometrischer Bedarf in der Synthese (20), gebildet wird, und dass zumindest in dem ersten Betriebsmodus in den Reformierungseinsatz, in die Reformierung (10) und/oder in den Syntheseeinsatz in Abhängigkeit von dem stöchiometrischen Bedarf in der Synthese (20) Wasserstoff zugegeben wird. Eine entsprechende Anlage ist ebenfalls Gegenstand der vorliegenden Erfindung.

## Beschreibung

Die Erfindung betrifft ein Verfahren zur Herstellung eines oder mehrerer Syntheseprodukte, insbesondere von Methanol, und eine entsprechende Anlage gemäß den jeweiligen Oberbegriffen der unabhängigen Patentansprüche.

### Stand der Technik

Methanol ist der einfachste Alkohol und einer der wichtigsten Grundstoffe der chemischen Industrie. Edukt der heterogen katalysierten Methanolsynthese ist Synthesegas, ein Gemisch aus Wasserstoff, Kohlenmonoxid und Kohlendioxid. Nach Ammoniak ist Methanol quantitativ das zweitgrößte Synthesegasprodukt.

Bei der Methanolsynthese aus Synthesegas laufen drei grundlegende Reaktionen ab, die in den Gleichungen (1) bis (3) angegeben sind, nämlich die Methanolbildung aus Kohlenmonoxid gemäß Gleichung (1), die Methanolbildung aus Kohlendioxid gemäß Gleichung (2) und die reverse Wassergas-Shiftreaktion gemäß Gleichung (3):

CO + 2 H2 ⇄ CH₃OH ΔH⁰_{300K} = -90,8 kJ/mol (1)

CO₂ + 3 H₂ ⇄ CH₃OH + H₂O ΔH⁰_{300K} = -49,2 kJ/mol (2)

CO₂ + H₂ ⇄ CO + H₂O ΔH⁰_{300K} = 41,2 kJ/mol (3)

Für die kommerzielle Synthesegasproduktion können prinzipiell alle kohlenstoffhaltigen Stoffe wie Kohle, Koks, Erdgas, Erdöl und Erdölfraktionen eingesetzt werden. Auch die Synthesegasproduktion aus Biomasse und Abfall ist grundsätzlich bekannt und kann im Rahmen der vorliegenden Erfindung eingesetzt werden. Aus wirtschaftlichen Gründen, wird in den meisten Methanolgroßanlagen Erdgas eingesetzt, weil dadurch Synthesegas am kostengünstigsten hergestellt werden kann.

Das entstehende Synthesegas wird durch die Stöchiometriezahl (SN) in Gleichung (4) charakterisiert. Dies Stöchiometriezahl bestimmt das Verhältnis der Konzentrationen von Wasserstoff, Kohlenmonoxid und Kohlendioxid. Für die Methanolsynthese sollte eine Stöchiometriezahl von etwas oberhalb von 2,0, insbesondere ca. 2,05, vorliegen. Ein Wasserstoffüberschuss führt zu einer Vergrößerung des Recyclestroms im Methanolprozess, da sich der Wasserstoff und die Inertgase darin anreichern. Ein Wasserstoffdefizit führt zu einer geringeren Methanolselektivität.

SN = ([H2) - [CO2]) / ([CO] + [CO2]) (4)

Dimethylether ist der strukturell einfachste Ether und fiel mit 3 bis 5 Gewichtsprozent lange Zeit als Nebenprodukt in der Hochdruck-Methanolsynthese an. Mit dem Übergang zur Niederdruck-Methanolsynthese wurden spezielle Anlagen zur Synthese von Dimethylether erbaut. Industriell ist Dimethylether als Ausgangsstoff für das Methylierungsmittel Dimethylsulfat und als Aerosoltreibgas von Bedeutung.

Die Synthese von Dimethylether ist grundsätzlich bekannt. Sie kann ein- oder zweistufig erfolgen. Aktueller Stand der Technik ist überwiegend die zweistufige Synthese, mit der Methanolsynthese als erster und der Dehydratisierung von Methanol zu Dimethylether als zweiter Stufe. Die einstufige Synthese fasst die Methanol- und Dimethylethersynthese in einem Reaktor zusammen.

Ein "einschrittiges" Verfahren zeichnet sich im hier verwendeten Sprachgebrauch insbesondere dadurch aus, dass keine Zwischenabtrennung oder eine entsprechende Ausschleusung eines methanolhaltigen Stoffstroms aus einem Reaktor und die Überführung in einen anderen Reaktor erfolgt, sondern dass insbesondere in demselben Katalysatorbett gebildetes Methanol zu Dimethylether weiterreagiert.

Zu Details sei auf einschlägige Fachliteratur, beispielsweise zur Methanolsynthese auf den Artikel "Methanol" in Ullmann's Encyclopedia of Industrial Chemistry, Onlineausgabe 15 Oktober 2012, und zur Dimethylethersynthese auf das "DME Handbook" des Japan DME Forum, 2007, oder die Artikel von T. Ogawa et al., "Direct Dimethyl Ether Synthesis", Journal of Natural Gas Chemistry 12, 2003, Seiten 219-227 bzw. S.-H. Lee et al., "Scale Up Study of DME Direct Synthesis Technology", Proceedings of the 24th World Gas Conference, Buenos Aires, 2009, beschrieben.

Wenngleich die vorliegende Erfindung nachfolgend mit dem Schwerpunkt der Methanol- und/oder Dimethylethersynthese beschrieben wird, kann diese grundsätzlich auch bei der Synthese anderer Syntheseprodukte zum Einsatz kommen. Mit anderen Worten können im Rahmen der vorliegenden Erfindung Methanol als Hauptprodukt, aber auch Dimethylether wie erläutert, Aldehyde und Alkohole (beispielsweise durch Hydroformylierung), Olefine und Paraffine (beispielsweise auf Grundlage von Fischer-Tropsch-Verfahren), Monoethylenglycol (beispielsweise aus Synthesegas) und höhere Alkohole erzeugt werden. Weitere Produkte können beispielsweise Essigsäure, Ameisensäure und Formaldehyd sein.

Die vorliegende Erfindung stellt sich die Aufgabe, Kohlendioxidemissionen bei der Herstellung von derartigen Syntheseprodukten zu verringern und dabei insbesondere extern bereitgestelltes Kohlendioxid verstärkt nutzbar zu machen. Ein weiteres Ziel ist die Nutzung fluktuierend bereitgestellten sogenannten "grünen" oder "blauen" Wasserstoffs, d.h. insbesondere von Wasserstoff, der durch Wasserelektrolyse unter Verwendung regenerativ erzeugten elektrischen Stroms erzeugt wird.

### Offenbarung der Erfindung

Vor diesem Hintergrund schlägt die Erfindung ein Verfahren zur Herstellung eines oder mehrerer Syntheseprodukte, insbesondere von Methanol, und eine entsprechende Anlage mit den jeweiligen Merkmalen der unabhängigen Patentansprüche vor. Bevorzugte Ausgestaltungen sind Gegenstand der abhängigen Patentansprüche sowie der nachfolgenden Beschreibung.

Die vorliegende Erfindung beruht auf der Erkenntnis, dass sich ein für die hier in Frage kommenden und zuvor erläuterten Synthesen, insbesondere die Methanolsynthese ein an sich schlechter geeignet erscheinendes Synthesegas bereitgestellt wird, wodurch aber ermöglicht wird, neben dem Import von Kohlendioxid in das Verfahren auch Wasserstoff zu importieren, der insbesondere regenerativ hergestellt werden kann. Auf diese Weise kann der Kohlendioxid-Fußabdruck eines entsprechenden Verfahrens deutlich reduziert werden. Dies gilt grundsätzlich für alle der erläuterten Verfahrensprodukte und die entsprechenden Verfahren zu deren Herstellung, insbesondere aber für die Methanolherstellung. Für die anderen Verfahrensprodukte und die entsprechenden Verfahren gelten die nachfolgenden Erläuterungen entsprechend bzw. können die nachfolgend erläuterten Zusammenhänge durch den Fachmann ohne weiteres übertragen werden. Ist daher nachfolgend von einer Synthese von Methanol und/oder Dimethylether die Rede, gelten die entsprechenden Erläuterungen für sämtliche anderen Verfahrensprodukte in gleicher Weise.

Wie erwähnt wird für die Methanolsynthese ein Synthesegas mit einer Stöchiometriezahl von etwas oberhalb von 2,0, insbesondere ca. 2,05, benötigt. Dies wird herkömmlicherweise durch die Verwendung einer Dampfreformierung (engl. Steam Methane Reforming, SMR) mit Kohlendioxidimport stromauf oder stromab des Reaktors, eine Partielle Oxidation (engl. Partial Oxidation, POX) mit Entfernung von Kohlendioxid aus einem Recycle zum Reaktor (z.B. umfassend die Verwendung von Membran- oder Druckwechseladsorptionsverfahren) oder beispielsweise das sogenannte Combined Reforming (CR) sichergestellt.

Bei der Dampfreformierung wird gemäß Gleichung (5) Erdgas mit Wasserdampf zu einem wasserstoffreichen Synthesegas umgesetzt. Bei der Partiellen Oxidation wird Sauerstoff eingesetzt wie aus Gleichung (6) ersichtlich. Die sogenannte Autotherme Reformierung (engl. Autothermal Reforming, ATR) stellt eine interne Kombination aus Dampfreformierung und Partieller Oxidation in einem Reaktor dar. Hierdurch können die Vorteile der Partiellen Oxidation (Bereitstellung thermischer Energie) und Dampfreformierung (hoher Wasserstoffgehalt) kombiniert werden. Das Combined Reforming verknüpft wiederum die beiden Verfahren Dampfreformierung und Autotherme Reformierung, jedoch in zwei getrennten Einheiten. Combined Reforming und Autotherme Reformierung haben den Vorteil, dass sie hinsichtlich des Verhältnisses von Wasserstoff zu Kohlenmonoxid sehr flexibel sind und das Synthesegas bereits unter erhöhtem Druck bereitgestellt wird.

CH₄ + H₂O ⇄ CO + 3 H₂ ΔH⁰_{298K} = 206 kJ/mol (5)

CH₄ + ½ O₂ ⇄ CO + 2 H₂ ΔH⁰_{298K} = -36 kJ/mol (6)

Alternativ ist auch die Methanolsynthese aus Wasserstoff und Kohlendioxid bekannt. Hierzu wird jedoch ein anderer Katalysator benötigt. Dampfreformierung, Partielle Oxidation, Autotherme Reformierung und Combined Reforming können gemäß der US 2015/0018592 A1 auch zur Direktsynthese von Dimethylether verwendet werden.

Die vorliegende Erfindung schlägt nun eine Synthesegasherstellung unter Verwendung eines auf Kohlendioxid und Erdgas basierenden Verfahrens, z.B. des sogenannten Dry Reformings (DryRef, ggf. auch mit einem gewissen Dampfanteil, auch als Bi-Reformierung bezeichnet), der Autothermen Reformierung oder der Partiellen Oxidation, jeweils mit Kohlendioxidimport, vor.

Beim Dry Reforming wird Erdgas mit Kohlendioxid nach Gleichung (7) zu einem kohlenmonoxidreichen Synthesegas umgesetzt. Dieses Verfahren ist insbesondere für die direkte Synthese von Dimethylether relevant.

CH₄ + CO₂ ⇄ 2 CO + 2 H₂ ΔH⁰_{298K} = 247 kJ/mol (7)

Die zuletzt genannten Verfahren führen zu Synthesegasen mit Stöchiometriezahlen unterhalb von 2, was grundsätzlich für die genannten Synthesezwecke nachteilig ist und entsprechende Verfahren daher auf den ersten Blick für eine entsprechende Synthese ungeeignet erscheinen lässt. Allerdings ermöglichen diese Verfahren eine Nutzung von Kohlendioxid durch einen entsprechenden Import. Die Stöchiometriezahl wird im Rahmen der vorliegenden Erfindung durch einen Wasserstoffimport, insbesondere aus einer Wasser- oder Chlor-Alkali-Elektrolyse, ausgeglichen.

Der wesentliche Vorteil gegenüber bekannten Verfahren ist damit der erhöhte Kohlendioxidimport und damit die geringeren Emissionen der das Kohlendioxid bildenden Verfahren. Dabei können konventionelle, für Synthesegasreaktionen eingerichtete Synthesereaktoren mit entsprechenden bewährten Katalysatoren und Trennteilen weiterverwendet werden. Dies wäre nicht der Fall, wenn nur Wasserstoff und Kohlendioxid als Einsatzstoffe vorliegen würden. Auf den ersten Blick erscheint es widersinnig, Synthesegas mit "falscher" Synthesegaszusammensetzung (d.h. mit zu geringem Wasserstoffgehalt zu bilden, wenn Erdgas zur Verfügung steht, das mit vier Wasserstoffatomen pro Kohlenstoffatom im Methanmolekül ausreichend Wasserstoff liefern kann. Die vorliegende Erfindung bietet jedoch in diesem Zusammenhang den Vorteil der Nachhaltigkeit.

Falls beispielsweise die direkte Synthese von Dimethylether aus einem Synthesegas mit einer Stöchiometriezahl von 2 erfolgen soll, wie dies grundsätzlich möglich ist, erfolgt die Synthesegaserzeugung wie soeben für das Beispiel Methanol erläutert. Eine Bereitstellung von Synthesegas mit einer Stöchiometriezahl von 1 für die direkte Synthese von Dimethylether gemäß einer anderen Ausgestaltung kann dagegen mit einem Synthesegas erfolgen, das aus der Dampfreformierung mit Kohlendioxidimport, einem Dry Reforming, einer Partiellen Oxidation bzw. Autothermen Reformierung, ggf. mit Kohlendioxidimport) und Kombinationen erfolgen, die direkt das Synthesegas erzeugen, dass im Make-Up für die direkte Synthese von Dimethylether benötigt wird.

Um die erfindungsgemäß angestrebte Verwendung von zusätzlichem Elektrolysewasserstoff zu ermöglichen, werden im Rahmen der vorliegenden Erfindung entsprechende Verfahren insbesondere auf eine hohe Kohlenmonoxidbildung optimiert. Die vorliegende Erfindung eignet sich dabei insbesondere auch im Zusammenhang mit Dry Reforming, das aufgrund seines sehr kohlenmonoxidreichen Synthesegases sich nur für eine begrenzte Anzahl von Verfahren eignet.

Die vorliegende Erfindung schlägt insgesamt ein Verfahren zur Herstellung eines oder mehrerer Syntheseprodukte, insbesondere von Methanol, vor, bei dem ein Methan und optional Kohlendioxid enthaltender Reformierungseinsatz unter Erhalt eines Synthesegases einer Reformierung unterworfen wird.

Der Methan enthaltende Reformierungseinsatz kann insbesondere mehr als 50, 60, 70, 80 oder 90 Volumenprozent Methan im Trockenanteil aufweisen und/oder vorteilhafterweise aus Erdgas gebildet sein. Zu den Vorteilen sei auf die obigen Erläuterungen verwiesen. Auch eine Reformierung bzw. Vergasung anderer Einsatzstoffe ist grundsätzlich möglich, wie eingangs bereits erläutert.

Unter einer "Reformierung" soll dabei im Rahmen der vorliegenden Erfindung grundsätzlich jedes katalytische Verfahren verstanden werden, bei dem gasförmige oder in gasförmigen Zustand überführte, kohlenstoffhaltige Verbindungen zu einem Synthesegas gemäß dem fachüblichen Verständnis umgesetzt werden. Es kann insbesondere eine (Dampf-)Reformierung mit Kohlendioxidzugabe (auch unter reduzierter Dampfzugabe, z.B. mit einem Verhältnis von Dampf zu Kohlenstoff von 0,2 bis 1,5 oder genauer 0,8 bis 1,5), eine Trockenreformierung (Dry Reforming, Umsetzung von Methan und Kohlendioxid praktisch ohne Dampf), eine autotherme Reformierung (insbesondere mit Kohlendioxidzugabe) und/oder eine partielle Oxidation (insbesondere mit Kohlendioxidzugabe) erfolgen.

Im Rahmen der vorliegenden Erfindung wird zumindest ein Teil des Synthesegases zur Bereitstellung eines Syntheseeinsatzes verwendet, und zumindest ein Teil des Syntheseeinsatzes wird unter Erhalt des einen oder der mehreren Syntheseprodukte einer Synthese unterworfen.

Erfindungsgemäß werden zumindest in einem ersten Betriebsmodus in den Reformierungseinsatz, in die Reformierung und/oder in den Syntheseeinsatz Kohlendioxid zugegeben und das Synthesegas wird zumindest in dem ersten Betriebsmodus in der Reformierung mit einem Wasserstoffgehalt, der geringer ist als ein stöchiometrischer Bedarf in der Synthese und mit einem Kohlenmonoxidgehalt, der größer ist als ein stöchiometrischer Bedarf in der Synthese, gebildet. Ferner wird zumindest in dem ersten Betriebsmodus in den Reformierungseinsatz, in die Reformierung und/oder in den Syntheseeinsatz in Abhängigkeit von dem stöchiometrischen Bedarf in der Synthese Wasserstoff zugegeben. Die Vorteile der Erfindung, die insbesondere in einer verbesserten Nutzung von Kohlendioxid und insbesondere regenerativ erzeugten Wasserstoffs bestehen, sei ebenfalls auf die obigen Erläuterungen verwiesen. Der "erste" Betriebsmodus kann auch der einzige Betriebsmodus des erfindungsgemäßen Verfahrens sein.

Das Synthesegas kann im Rahmen der vorliegenden Erfindung insbesondere mit einem Verhältnis von Wasserstoff zu Kohlendioxid von 0,5 bis 3, insbesondere von 0,8 bis 2, weiter insbesondere von 1 bis 1,5, und/oder mit einer Stöchiometriezahl von 0,5 bis 3, insbesondere von 0,8 bis 2, weiter insbesondere von 1 bis 1,5, bereitgestellt werden. Die Stöchiometriezahl das Verhältnis von Wasserstoff zu Kohlendioxid können in denselben Wertebereichen bzw. bei denselben Werten liegen, wenn Kohlendioxid aus dem Synthesegas abgetrennt wird

Die Synthese kann im Rahmen der vorliegenden Erfindung, wie erwähnt, insbesondere eine Methanolsynthese, eine Dimethylethersynthese und/oder eine Fischer-Tropsch-Synthese unter Bereitstellung des Produkts umfassen. Wie einleitend erwähnt und hier nur der Vollständigkeit nochmals erwähnt, können im Rahmen der Erfindung Methanol als Hauptprodukt, aber auch Dimethylether (insbesondere in einer einstufigen Dimethylethersynthese), Aldehyde und Alkohole (beispielsweise durch Hydroformylierung), Olefine und Paraffine (beispielsweise auf Grundlage von Fischer-Tropsch-Verfahren), Monoethylenglycol (beispielsweise aus Synthesegas) und höhere Alkohole erzeugt werden. Weitere Produkte können beispielsweise Essigsäure, Ameisensäure und Formaldehyd sein. Alle diese Verbindungen können ein Syntheseprodukt im Sinne der vorliegenden Erfindung darstellen bzw. in Gemischen gebildet werden, die mehrere solcher Syntheseprodukte umfassen. Die im Rahmen der vorliegenden Erfindung eingesetzten Synthesen können sämtliche zur Herstellung entsprechender Verbindungen und im Zusammenhang mit den erfindungsgemäß vorgeschlagenen Maßnahmen vorteilhaften Verfahren darstellen.

Der Wasserstoff kann im Rahmen der vorliegenden Erfindung insbesondere mittels Wasser- und/oder Chlor-Alkali-Elektrolyse bereitgestellt werden, wobei sich jedes beliebige Elektrolyseverfahren eignet.

Bei der klassischen Wasserelektrolyse wird eine wässrige alkalische Lösung, typischerweise von Kaliumhydroxid, als Elektrolyt verwendet (AEL, Alkalische Elektrolyse). Die Elektrolyse mit einer uni- oder bipolaren Elektrodenanordnung erfolgt dabei bei Atmosphärendruck oder im industriellen Maßstab auch bei einem Druck von bis zu 30 bar. Neuere Entwicklungen bei der Wasserelektrolyse umfassen die Verwendung von protonenleitenden lonenaustauschmembranen (SPE, Solid Polymer Electrolysis; PEM, Proton Exchange Membranes), bei der das zu elektrolysierende Wasser an der Anodenseite bereitgestellt wird. Die genannten Verfahren zählen zu den Niedertemperaturverfahren, bei denen das zu elektrolysierende Wasser in der Flüssigphase vorliegt. Daneben wird auch die sogenannte Dampfelektrolyse vorgenommen, die ebenfalls mit alkalischen Elektrolyten (also als AEL) mit angepassten Membranen, beispielsweise Polysulfonmembranen, sowie unter Verwendung von Festoxidelektrolysezellen (SOEC, Solid Oxide Electrolysis Cells) durchgeführt werden können. Letztere umfassen insbesondere dotiertes Zirkondioxid oder Oxide anderer seltener Erden, die bei mehr als 800 °C leitfähig werden. Der Begriff der Wasserelektrolyse soll nachfolgend sämtliche dieser Verfahren umfassen.

In einer besonders bevorzugten Ausgestaltung des Verfahrens wird in dem ersten Betriebsmodus eine Menge des in den Reformierungseinsatz, die Reformierung und/oder den Syntheseeinsatz zugegebenen Kohlendioxids und eine Menge des in den Reformierungseinsatz, in die Reformierung und/oder in den Syntheseeinsatz zugegebenen Wasserstoffs verändert. Auf diese Weise kann ein Verfahren geschaffen werden, das in Anpassung an ein Wasserstoffangebot, das sich insbesondere nach der verfügbaren Menge regenerativ erzeugter elektrischer Energie richten kann, flexibel betreibbar ist und auf diese Weise ein "Peak Shaving" durchführen kann. Wird weniger Kohlendioxid zugegeben, sinkt auch der Bedarf nach Wasserstoff.

In einer Ausgestaltung kann dabei in einem zweiten Betriebsmodus in den Reformierungseinsatz, die Reformierung und/oder den Syntheseeinsatz kein Kohlendioxid oder weniger Kohlendioxid als in dem Betriebsmodus zugegeben werden, und in dem zweiten Betriebsmodus kann entsprechend in den Reformierungseinsatz, in die Reformierung und/oder in den Syntheseeinsatz kein Wasserstoff oder weniger Wasserstoff als in dem ersten Betriebsmodus zugegeben werden.

In einer vorteilhaften Ausgestaltung des erfindungsgemäßen Verfahrens wird in der Synthese ein das Produkt und nicht umgesetzte Komponenten des Syntheseeinsatzes enthaltendes Produktgemisch gebildet wird und zumindest ein Teil des Produktgemischs wird einer Trennung unterworfen.

Die nicht umgesetzten Komponenten des Syntheseeinsatzes können dabei insbesondere Wasserstoff und/oder Kohlenmonoxid umfassen, die in der Trennung in eine erste Rückführfraktion überführt werden, wobei die erste Rückführfraktion zumindest teilweise in den Syntheseeinsatz zurückgeführt wird. Auf diese Weise lässt sich die Ausbeute des erfindungsgemäßen Verfahrens erhöhen.

Die nicht umgesetzten Komponenten des Syntheseeinsatzes können ferner Kohlendioxid umfassen, das in der Trennung in eine zweite Rückführfraktion überführt wird, wobei die zweite Rückführfraktion zumindest teilweise in den Reformierungseinsatz, in die Reformierung und/oder in den Syntheseeinsatz zurückgeführt wird. Diese Rückführmenge wird insbesondere mit der zuzuspeisenden Menge an frischem Kohlendioxid abgeglichen.

Im Rahmen der vorliegenden Erfindung kann in der Trennung ferner ein Zwischenprodukt der Synthese, im Fall einer Dimethylethersynthese insbesondere Methanol, in einer Zwischenproduktfraktion abgetrennt werden, wobei zumindest ein Teil der Zwischenproduktfraktion in die Synthese zurückgeführt wird.

Die Erfindung erstreckt sich ferner auf eine Anlage zur Herstellung eines Produkts, mit Mitteln, die dafür eingerichtet sind, einen Methan enthaltenden Reformierungseinsatz unter Erhalt eines Synthesegases einer Reformierung zu unterwerfen, zumindest einen Teil des Synthesegases zur Bereitstellung eines Syntheseeinsatzes zu verwenden, und zumindest einen Teil des Syntheseeinsatzes unter Erhalt des Produkts einer Synthese zu unterwerfen.

Die Anlage zeichnet sich aus durch Mittel, die dafür eingerichtet sind, zumindest in einem ersten Betriebsmodus in den Reformierungseinsatz, die Reformierung und/oder den Syntheseeinsatz Kohlendioxid zuzugeben, zumindest in dem ersten Betriebsmodus das Synthesegas in der Reformierung mit einem Wasserstoffgehalt, der geringer ist als ein stöchiometrischer Bedarf in der Synthese und mit einem Kohlenmonoxidgehalt, der größer ist als ein stöchiometrischer Bedarf in der Synthese, zu bilden, und zumindest in dem ersten Betriebsmodus in den Reformierungseinsatz, in die Reformierung und/oder in den Syntheseeinsatz in Abhängigkeit von dem stöchiometrischen Bedarf in der Synthese Wasserstoff zuzugeben.

Zu Merkmalen und Vorteilen der erfindungsgemäß vorgeschlagenen Anlage sei ausdrücklich auf die obigen Erläuterungen bezüglich des erfindungsgemäßen Verfahrens und seiner Ausgestaltungen verwiesen. Dies gilt auch für eine Anlage gemäß einer besonders bevorzugten Ausführungsform der vorliegenden Erfindung, die zur Durchführung eines Verfahrens eingerichtet ist, wie es zuvor in seinen Ausgestaltungen erläutert wurde.

Die Erfindung wird nachfolgend unter Bezugnahme auf die beigefügten Zeichnungen, welche bevorzugte Ausgestaltungen der vorliegenden Erfindung veranschaulichen, näher erläutert.

### Kurze Beschreibung der Zeichnungen

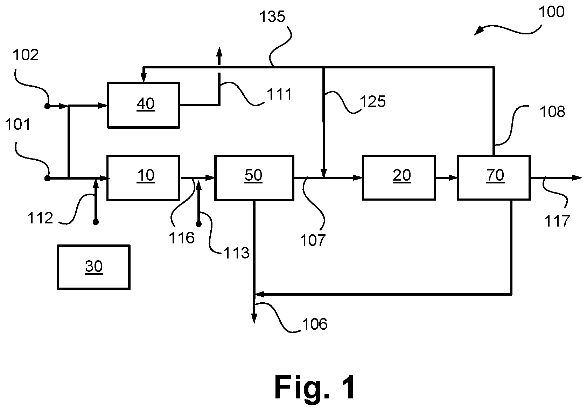

Figur 1 veranschaulicht ein Verfahren gemäß einer Ausführungsform der Erfindung.

### Ausführliche Beschreibung der Zeichnungen

In den Figuren sind jeweils Verfahren gemäß Ausgestaltungen der Erfindung veranschaulicht. Die Erläuterungen gelten dabei für entsprechende Anlagen in gleicher Weise. Baulich oder funktionell einander entsprechende Anlagenteile bzw. Verfahrensschritte sind dabei jeweils mit identischen Bezugszeichen angegeben und werden lediglich der Übersichtlichkeit halber nicht wiederholt erläutert.

In Figur 1 ist ein Verfahren zur Herstellung Methanol gemäß einer Ausgestaltung der vorliegenden Erfindung veranschaulicht und insgesamt mit 100 bezeichnet. Wesentliche Verfahrensschritte sind hierbei eine Reformierung 10, beispielsweise in Form eines Dry Reforming, und eine Synthese 20, die hier in Form einer Methanolsynthese ausgebildet ist. Ferner sind hier eine Elektrolyse 30, eine Verbrennung 40, eine Wärmerückgewinnung 50 und eine Trennung 70 vorhanden. Auch andere Reformierungsverfahren können hier in entsprechender Weise zum Einsatz kommen und die Erfindung kann sich auch auf andere Verfahrensprodukte beziehen, wie mehrfach erläutert.

Dem Verfahren 100 werden im dargestellten Beispiel Erdgas 101 bzw. allgemeiner ein methanhaltiger Reformierungseinsatz und Verbrennungsluft 102 von der Anlagengrenze zugeführt. Das Erdgas 101 wird auf die Reformierung 10 und den Brenner 30 aufgeteilt, die Luft 102 dem Brenner zugeführt. In dem Verfahren 100 werden ein Kohlendioxidstrom 112 und ein Wasserstoffstrom 113, beispielsweise aus einer entsprechenden Elektrolyse 30, separat vor bzw. nach der Trockenreformierung 10 zugegeben eingespeist. Auf diese Weise weist ein gebildetes Synthesegas 116 insbesondere einen geringeren Gehalt an Methan auf als ein Synthesegas, das ohne eine entsprechende Zugabe bereitgestellt würde. In die Reformierung 10 können in einer anderen Ausgestaltung Kohlendioxid und Wasserstoff aber auch gemeinsam in Form eines Stoffstroms 103 eingespeist werden. In beiden Fällen können die zuvor erwähnten Vorteile, nämlich der Verbrauch von Kohlendioxid und die Nutzung von Wasserstoff, der insbesondere regenerativ bereitgestellt werden kann, erzielt werden. Das gebildete Synthesegas 116 weist für die Methanolsynthese insbesondere eine Stöchiometriezahl von 2 bis 2,05 auf, was insbesondere auch durch die Wasserstoffzugabe erzielt wird. Das Synthesegas wird nach der Kühlung in der Wärmerückgewinnung 50 der Synthese 20 zugeführt. Nicht umgesetzte Synthesegaskomponenten wie Wasserstoff und Kohlenmonoxid werden in der Trennung 70 in Form eines Stoffstroms 108 abgetrennt. Ein Teil des Stoffstroms 108 kann, wie hier in Form eines Stoffstroms 125 dargestellt, in den Syntheseeinsatz stromauf der Synthese 20 geführt werden. Ein weiterer Teil kann in Form eines Stoffstroms 135 auch in die Verbrennung 40 geführt und dort zu einem Verbrennungsabgas 111 umgesetzt werden.

Im Beispiel werden in der Wärmerückgewinnung 50 und in der Trennung 70 jeweils Wasserströme abgetrennt und in Form eines Sammelstroms 106 aus dem Verfahren 100 ausgeleitet. Aus der Wärmerückgewinnung 50 ausgeführtes Synthesegas 107 wird mit dem Stoffstrom 125 und mit in der Trennung 60 ebenfalls erhaltendem Methanol 109 vereinigt, bevor es in die Synthese 20 eingespeist wird. In der Synthese 20 wird außerdem ein Methanolstrom 110 gebildet.

Es versteht sich, dass alle isoliert in Bezug auf bestimmte Figuren bzw. Ausführungsbeispiele beschriebenen Merkmale auch bei anderen Ausführungsbeispielen, falls in Kombination beschrieben alleine, oder falls alleine beschrieben in Kombination, eingesetzt werden können.

## Patentansprüche

1. Verfahren (100) zur Herstellung eines oder mehrerer Syntheseprodukte, bei dem ein Methan enthaltender Reformierungseinsatz unter Erhalt eines Synthesegases einer Reformierung (10) unterworfen wird, bei dem zumindest ein Teil des Synthesegases zur Bereitstellung eines Syntheseeinsatzes verwendet wird, und bei dem zumindest ein Teil des Syntheseeinsatzes unter Erhalt des Produkts einer Synthese (20) unterworfen wird, **dadurch gekennzeichnet, dass** zumindest in einem ersten Betriebsmodus in den Reformierungseinsatz, die Reformierung (10) und/oder den Syntheseeinsatz Kohlendioxid zugegeben wird, dass zumindest in dem ersten Betriebsmodus das Synthesegas in der Reformierung (10) mit einem Wasserstoffgehalt, der geringer ist als ein stöchiometrischer Bedarf in der Synthese (20) und mit einem Kohlenmonoxidgehalt, der größer ist als ein stöchiometrischer Bedarf in der Synthese (20), gebildet wird, und dass zumindest in dem ersten Betriebsmodus in den Reformierungseinsatz, in die Reformierung (10) und/oder in den Syntheseeinsatz in Abhängigkeit von dem stöchiometrischen Bedarf in der Synthese (20) Wasserstoff zugegeben wird.

2. Verfahren (100) nach Anspruch 1, bei dem der Reformierungseinsatz Kohlendioxid enthält, das dem Reformierungseinsatz zugegeben wird.

3. Verfahren (100) nach Anspruch 1 oder 2, bei dem das Synthesegas in dem ersten Betriebsmodus mit einem Verhältnis von Wasserstoff zu Kohlendioxid von 0,5 bis 3 und/oder mit einer Stöchiometriezahl von 0,5 - 3 bereitgestellt wird.

4. Verfahren (100) nach einem der vorstehenden Ansprüche, bei dem die Synthese (20) eine Methanolsynthese, eine einstufige Dimethylethersynthese, eine Synthese eines oder mehrerer Aldehyde, eine Synthese eines oder mehrerer Olefine und/oder Paraffine, eine Synthese von Monoethylenglycol, eine Synthese eines oder mehrerer höherer Alkohole, eine Essigsäuresynthese, eine Ameisensäuresynthese, eine Formaldehydsynthese oder eine Kombination zweier oder mehrerer beliebiger solcher Synthesen umfasst.

5. Verfahren (100) nach einem der vorstehenden Ansprüche, bei dem der Wasserstoff mittels Wasser- und/oder Chlor-Alkali-Elektrolyse bereitgestellt wird.

6. Verfahren (100) nach einem der vorstehenden Ansprüche, bei dem der Methan enthaltende Reformierungseinsatz mehr als 50, 60, 70, 80 oder 90 Volumenprozent Methan im Trockenanteil aufweist und/oder bei dem der Methan enthaltende Reformierungseinsatz aus Erdgas gebildet ist.

7. Verfahren (100) nach einem der vorstehenden Ansprüche, bei dem in dem ersten Betriebsmodus eine Menge des in den Reformierungseinsatz, die Reformierung (10) und/oder den Syntheseeinsatz zugegebenen Kohlendioxids und eine Menge des in den Reformierungseinsatz, in die Reformierung (10) und/oder in den Syntheseeinsatz zugegebenen Wasserstoffs verändert werden.

8. Verfahren (100) nach einem der vorstehenden Ansprüchen, bei dem in einem zweiten Betriebsmodus in den Reformierungseinsatz, die Reformierung (10) und/oder den Syntheseeinsatz kein Kohlendioxid oder weniger Kohlendioxid als in dem Betriebsmodus zugegeben wird, und dass in dem zweiten Betriebsmodus in den Reformierungseinsatz, in die Reformierung (10) und/oder in den Syntheseeinsatz kein Wasserstoff oder weniger Wasserstoff als in dem ersten Betriebsmodus zugegeben wird.

9. Verfahren (100) nach einem der vorstehenden Ansprüche, bei dem in der Synthese (20) ein das Produkt und nicht umgesetzte Komponenten des Syntheseeinsatzes enthaltendes Produktgemisch gebildet wird und zumindest ein Teil des Produktgemischs einer Trennung (70) unterworfen wird.

10. Verfahren (100, 200, 300) nach Anspruch 9, bei dem die nicht umgesetzten Komponenten des Syntheseeinsatzes Wasserstoff und/oder Kohlenmonoxid umfassen, die in der Trennung (60) in eine erste Rückführfraktion überführt werden, wobei die erste Rückführfraktion zumindest teilweise in den Syntheseeinsatz zurückgeführt wird.

11. Verfahren (100) nach Anspruch 9 oder 10, bei dem die nicht umgesetzten Komponenten des Syntheseeinsatzes Kohlendioxid umfassen, das in der Trennung (70) in eine zweite Rückführfraktion überführt wird, wobei die zweite Rückführfraktion zumindest teilweise in den Reformierungseinsatz, in die Reformierung (10) und/oder in den Syntheseeinsatz zurückgeführt wird.

12. Verfahren (100) nach einem der Ansprüche 9 bis 11, bei dem in der Trennung (60) ein Zwischenprodukt der Synthese (20, 70) in einer Zwischenproduktfraktion abgetrennt wird, wobei zumindest ein Teil der Zwischenproduktfraktion in die Synthese (20, 70) zurückgeführt wird.

13. Anlage zur Herstellung eines oder mehrerer Syntheseprodukte, mit Mitteln, die dafür eingerichtet sind, einen Methan enthaltenden Reformierungseinsatz unter Erhalt eines Synthesegases einer Reformierung (10) zu unterwerfen, zumindest einen Teil des Synthesegases zur Bereitstellung eines Syntheseeinsatzes zu verwenden, und zumindest einen Teil des Syntheseeinsatzes unter Erhalt des Produkts einer Synthese (20, 70) zu unterwerfen, **gekennzeichnet durch** Mittel, die dafür eingerichtet sind, zumindest in einem ersten Betriebsmodus in den Reformierungseinsatz, die Reformierung (10) und/oder den Syntheseeinsatz Kohlendioxid zuzugeben, zumindest in dem ersten Betriebsmodus das Synthesegas in der Reformierung (10) mit einem Wasserstoffgehalt, der geringer ist als ein stöchiometrischer Bedarf in der Synthese (20, 70) und mit einem Kohlenmonoxidgehalt, der größer ist als ein stöchiometrischer Bedarf in der Synthese (20, 70), zu bilden, und zumindest in dem ersten Betriebsmodus in den Reformierungseinsatz, in die Reformierung (10) und/oder in den Syntheseeinsatz in Abhängigkeit von dem stöchiometrischen Bedarf in der Synthese (20, 70) Wasserstoff zuzugeben.

14. Anlage nach Anspruch 13, die zur Durchführung eines Verfahrens nach einem der Ansprüche 1 bis 12 eingerichtet ist.
